# EUROPEAN PATENT SPECIFICATION

(11) **EP 2 937 077 B1**
(45) Date of publication and mention of the grant of the patent: **06.10.2021**
(21) Application number: 15164590.0
(22) Date of filing: 22.04.2015
(51) Int. Cl.: A61K 31/4375, A61K 9/08, A61K 36/185, A61K 36/29, A61K 36/00, A61P 11/04

(54) **ORAL LIQUID FORMULATIONS COMPRISING HERBAL EXTRACTS**
ORALE FLÜSSIGE FORMULIERUNGEN MIT KRÄUTEREXTRAKTEN
FORMULATIONS ORALES LIQUIDES COMPRENANT DES EXTRAITS D'HERBES

(30) Priority: 24.04.2014 TR 201404654
(43) Date of publication of application: 28.10.2015
(73) Proprietor: MONTERO GIDA SANAYI VE TICARET A.S., Istinye, Istanbul 34460 (TR)
(72) Inventor: Cifter, Ümit, 34460 Istanbul (TR); Türkyilmaz, Ali, 34460 Istanbul (TR); Hatirnaz, Zekiye Basak, 34460 Istanbul (TR); Yelken, Gülay, 34460 Istanbul (TR); Zan, Merve, 34460 Istanbul (TR)
(74) Representative: Mutlu, Aydin

(56) References cited:
- EP-A1- 2 991 624
- DATABASE WPI Week 200969 Thomson Scientific, London, GB; AN 2009-P07060 XP002731321, & JP 2009 215276 A (FUJI FILM CO LTD) 24 September 2009 (2009-09-24)
- DATABASE WPI Week 200115 Thomson Scientific, London, GB; AN 2001-138918 XP002731322, & CN 1 147 948 A (CHINA CHEM PHARM CO LTD) 23 April 1997 (1997-04-23)
- DATABASE WPI Week 201437 Thomson Scientific, London, GB; AN 2014-G94896 XP002731323, & KR 2014 0044642 A (KUHNIL PHARM CO LTD) 15 April 2014 (2014-04-15)
- Dónal P. O'Mathúna: "Goldenseal: The Golden Cure for Common Colds?", Relias, Formely AHC Media , 1 August 1998 (1998-08-01), pages 1-6, Retrieved from the Internet: URL:https://www.ahcmedia.com/articles/prin t/38664-goldenseal-the-golden-cure-for-com mon-colds [retrieved on 2018-03-31]
- Dr Diehl: "Goldenseal: The Golden Cure for Common Colds?", , 1 August 1998 (1998-08-01), pages 1-5, XP055731686, Retrieved from the Internet: URL:https://www.reliasmedia.com/articles/p rint/38664-goldenseal-the-golden-cure-for- common-colds [retrieved on 2020-09-17]
- K. Ramanauskiene ET AL: "Propolis oil extract: quality analysis and evaluation of its antimicrobial activity", NATURAL PRODUCT RESEARCH, vol. 25, no. 15, 1 September 2011 (2011-09-01), pages 1463-1468, XP055731692, GB ISSN: 1478-6419, DOI: 10.1080/14786419.2010.529440

## Description

### Technical Field

The present invention relates to an herbal oral liquid pharmaceutical composition comprising berberine or a pharmaceutically acceptable salt form, pelargonium sidoides, and propolis, for providing treatment in various respiratory diseases such as cold cough, allergic asthma, seasonal allergic rhinitis, bronchitis, pharyngitis, laryngitis and pneumonitis.

### Background of the Invention

Berberine or 6-dihydro-9,10-dimethoxybenzo(g)-1,3-benzodioxolo-(5,6-a)-quinolizinium is an isoquinoline alkaloid present in herb plants. Berberine is obtained from various medicinal plants used in Chinese medicine such as Coptidis rhizoma, phellodenron, *Scutellaria baicalensis, Mahonia aquifolium* and berberis. According to the study of Dr. Diehl et al. (Donal P. O'Mathûna "Goldenseal: The Golden Cure for Common Colds?" 1 August 1998, pages 1-5), it has been known that Berberine is found in a number of other herbs with antimicrobial reputations used as stomach tonics, and Goldenseal is known for the treatment of common colds. Berberine is odorless but with a bitter taste powder which is sparingly soluble in methanol, slightly soluble in ethanol and very slightly soluble in water.

In prior art, berberine is generally formulated in solid oral dosage forms. However, the level of its solubility influences its absorption and bioavailability when it is formulated in solid dosage forms. To provide a good efficacy, it is important to formulate berberine in a dosage form wherein it is soluble, bioavailable and also stable. Also, in prior art it is already known that it is advantageous to use together several herbal extract to get better efficacy and also bioavailability.

In syrup formulations, therapeutic agent is dissolved and is therefore immediately available for absorption. Therefore, the bioavailability of the agent is greater than that of oral solid dosage forms. Syrup formulations are also advantageous because they are easy to administer and suitable for certain patient populations, for example children or adults who are unable to swallow solid oral dosage forms or use an inhaler device. However, as well as advantages, syrup formulations have their own problems like adjustement of solubility, especially during the process. Designing of oral herbal liquid formulation is a challenge. The poor solubility of the herbal extract may prohibit their formulation as pharmaceutical solutions. Furthermore, final preparation must satisfy the requirements of taste, appearence and viscosity.

In prior art, the patent application JP 2009 215276 discloses oral compositions containing a plant of the genus Salacia. This oral composition includes an extract of a plant belonging to the genus Salacia and at least one kind of a component having an anti-constipation effect or a component having anti-diarrhea effect.

CN 1147948 relates to a traditional medical preparation for relieving swelling and alleviating pain of throat.

KR 2014 0044642 relates to syrup formulations comprising Pelargonium sidoides extract and levodropropizine. Main purpose of the invention is to develop a pharmaceutical composition in stable syrup form without the formation of precipitates. Stability problem is solved by adjusting pH value.

EP 2991624 A1 mainly relates to oral solution formulations comprising Coptis rhizome and Pelargonium sidoides. Purpose of the invention is to prevent bitter taste of the formulation comprising the Coptis rhizome extract.

Even if some studies exist in prior art to formulate herbal extracts in liquid oral dosage forms, there still exists a need for new stable syrup formulations of herbal extracts preferably berberine for the treatment of respiratory diseases. Also, it is a big challenge to provide a liquid pharmaceutical composition comprising more than one herbal extract wherein it is more difficult to provide its level of solubility and stability.

### Objects of the Invention

The main object of the present invention is to obtain an oral liquid pharmaceutical composition comprising berberine or pharmaceutically acceptable salt form as an active agent, pelargonium sidoides and propolis.

Another main object of the present invention is to obtain an oral liquid pharmaceutical composition comprising berberine as an active agent, pelargonium sidoides and propolis, and at least one more herbal extract selected from the group comprising ginger, ginseng, licorice, echinacea, ginkgo biloba, turmeric, capsicum, sage, rosemary, maca, rhodiola and hedera helix.

Yet another object of the present invention is to develop an oral liquid pharmaceutical composition which is capable of providing an easy administration accompanied by an increased patient compliance and convenience.

Another object of the present invention is to provide an oral liquid pharmaceutical composition wherein a significantly improved pharmaceutical stability, as well as the excellent long-term stability is shown.

There is also provided an oral liquid pharmaceutical composition wherein the active ingredient is dissolved properly in liquid composition. Thus, stability of active ingredient and of liquid composition in which active ingredient is dissolved, can be secured.

### Detailed Description of the Invention

In order to achieve the aforementioned objects, the present invention discloses a novel, stable, bioavailable, liquid pharmaceutical composition for the oral administration of berberine as the active ingredient comprising one or more pharmaceutically acceptable excipient.

The term "berberine" as used herein refers to berberine as well as pharmaceutically acceptable salts, enantiomers, hydrates, solvates, metabolites, prodrugs and mixture thereof. The term also includes all polymorphic forms, whether crystalline or amorphous. According to the present invention, the preferred form of berberine is berberine hydrochloride.

According to another embodiment of the present invention, said oral liquid pharmaceutical composition can further include another herbal extract such as ginger, ginseng, licorice, echinacea, ginkgo biloba, turmeric, capsicum, sage, rosemary, maca, rhodiola and hedera helix. Said another herbal extract is preferably selected from the group comprising ginger, ginseng, licorice, echinacea and hedera helix.

According to an embodiment of the present invention, said oral liquid pharmaceutical composition comprises a) berberine or a pharmaceutically acceptable salt form, b) pelargonium sidoides, c) propolis and d) at least one pharmaceutically acceptable excipient.

According to an embodiment of the present invention, the oral liquid pharmaceutical composition comprises at least one pharmaceutically acceptable excipient which can be a diluent. Diluent can be selected from the group comprising, but not limited to; water, dimethylisosorbide, glycols, propylene glycol, ethyl alcohol, cetyl alcohol, glyceryl stearate, isopropyl alcohol, diethylamine, glyceryl oleate, myristyl alcohol, gelatin, simple syrup, cyclodextrin, polyvinyl pyrrolidone (Povidone), benzyl alcohol, glycerin, propylene glycol, ethanol, maltitol, xylitol, inositol, mannitol, invert sugars, sorbitol and the like or combination thereof. Said diluent is preferably selected from the group comprising glycerin, propylene glycol, sorbitol, water, ethanol and combination thereof.

According to a preferred embodiment of the present invention, the oral liquid pharmaceutical composition comprising a) berberine or a pharmaceutically acceptable salt form, b) pelargonium sidoides and c) at least two diluents. The use of at least two diluent according to the present invention enhance the solubility of herbal extract to the required level. In this direction, we have surprisingly found that the most suitable diluent system for the given purpose is the combination of propylene glycol and glycerin.

In syrup formulations, water is preferred as diluents and cosolvents due to the lack of toxicity. However, because of the slight solubility of herbal extracts, other diluents could be more effective to formulate them in liquid compositions. Therefore, according to the present invention, diluents such as propylene glycol and glycerin are used in oral liquid formulation. However, use of diluent system needs to consider the toxicity. In order to decrease or prevent the toxicity, the range of concentrations of each diluent should be arranged. Accordingly, we have found that the range of total diluent concentration of about 50 mg/mL to 900 mg/mL used in the present invention enhance the solubility of herbal extract and as well as limit the toxicity caused by diluents. The range of total diluent concentration can also be of about 100 mg/mL to 800 mg/mL.

According to an embodiment of the present invention, the oral liquid pharmaceutical composition a) berberine or a pharmaceutically acceptable salt form, b) pelargonium sidoides and c) at least two diluents wherein the total concentration of diluents is about 50 mg/mL to 900 mg/mL.

According to an embodiment of the present invention, the oral liquid pharmaceutical composition comprising a) berberine or a pharmaceutically acceptable salt form, b) pelargonium sidoides and c) the combination of propylene glycol and glycerin wherein the ratio of propylene glycol to glycerin is about 0.5:1 to 1:2. In prior art, propylene glycol is generally used as replacement for glycerin. But, the combination of propylene glycol and glycerin in the ratio according to the present invention facilitate the administration of the oral liquid formulation and enhance the solubility of berberine extract.

According to an embodiment of the present invention, the oral liquid pharmaceutical composition may further comprise one more diluent such as sorbitol which can be found in a concentration of 50 mg/mL to 250 mg/mL.

According to another embodiment of the present invention, the oral liquid pharmaceutical composition comprises one or more sweetening agent to enhances the taste of the composition. Sweetening agent used is selected from the group comprising, but not limited to, saccharine, saccharine sodium, sucralose, sorbitol, acesulfame potassium, aspartame, a fluorinated sucrose derivative, dextrose, corn syrup, alitame, cyclamic acid, thaumatin, monellin, mono-ammonium glycyrrhizinate and the like or combination therof. The most preferred sweetening agent of the present invention can be sorbitol, mono-ammonium glycyrrhizinate, sucralose or combination thereof.

Buffer system according to the present invention include preferably sodium citrate, potassium citrate, sodium citrate dihydrate, citric acid, citric acid monohydrate, sodium bicarbonate, potassium bicarbonate, sodium dihydrogen phosphate and potassium dihydrogen phosphate and the like or combination thereof. The most preferred buffer system of the present invention contains the combination of sodium citrate dihydrate and citric acid monohydrate. The concentration of buffer system used may be in the range of between 0.1 and 10 mg/mL and preferably between 0.5 and 5 mg/mL. Thus, control of the pH in the formulation is achieved using a buffer that does not adversely affect the solubility of herbal extract. Besides, pH control is performed to maintain the solubility and to enhance the stability of herbal extract in the syrup formulation.

Flavoring agent used according to the present invention is selected from the group comprising but not limited to, eucalytus, orange, peppermint, spearmint, lime, apple, pear, peach, raspberry, plum, pineapple flavour and the like or combination thereof. The concentration of flavoring agent used may be in the range of between 0.1 and 2 mg/mL and preferably between 0.1 and 3 mg/mL.

According to an embodiment of the present invention, said oral liquid pharmaceutical composition comprises;
a) berberine as an active agent, pelargonium sidoides, propolis,
b) optionally at least one more herbal extract,
c) at least two diluent,
d) at least one sweetening agent,
e) at least one pH buffer system, and
f) at least one flavoring agent.

According to an embodiment of the present invention said oral liquid pharmaceutical composition comprises;
a) berberine as an active agent, pelargonium sidoides, propolis,
b) optionally at least one more herbal extract selected from the group comprising ginger, ginseng, licorice, echinacea, hedera helix and combination thereof,
c) the combination of propylene glycol and glycerin and optionally sorbitol as diluent,
d) mono-ammonium glycyrrhizinate and sucralose as sweetening agent,
e) the combination of sodium citrate dihydrate and citric acid monohydrate as pH buffer system, and
f) eucalyptus as flavoring agent.

According to another embodiment of the present invention, said oral liquid pharmaceutical composition can further include at least one more herbal extract such as ginger, ginseng, licorice, echinacea, ginkgo biloba, turmeric, capsicum, sage, rosemary, maca, rhodiola and hedera helix. Said another herbal extract is preferably selected from the group comprising ginger, ginseng, licorice, echinacea and hedera helix.

## Claims

1. An oral liquid pharmaceutical composition comprising berberine or a pharmaceutically acceptable salt form, pelargonium sidoides, propolis and at least one pharmaceutically acceptable excipient.

2. The oral liquid pharmaceutical composition according to claim 1, wherein the pharmaceutically acceptable excipient is diluent.

3. The oral liquid pharmaceutical composition according to claim 2, wherein the total concentration of diluents is about 50 mg/mL to 900 mg/mL.

4. The oral liquid pharmaceutical composition according to claim 3, wherein the total concentration of diluents is about 100 mg/mL to 800 mg/mL.

5. The oral liquid pharmaceutical composition according to any preceding claims, wherein the diluent is selected from the group comprising water, dimethylisosorbide, glycols, propylene glycol, ethyl alcohol, cetyl alcohol, glyceryl stearate, isopropyl alcohol, diethylamine, glyceryl oleate, myristyl alcohol, gelatin, cyclodextrin, polyvinyl pyrrolidone (Povidone), benzyl alcohol, glycerin, propylene glycol, ethanol, maltitol, xylitol, inositol, mannitol, invert sugars, sorbitol and combination thereof.

6. The oral liquid pharmaceutical composition according to claim 5, wherein the diluent is selected from the group comprising glycerin, propylene glycol, sorbitol, water, ethanol and combination thereof.

7. The oral liquid pharmaceutical composition according to any preceding claims, comprises at least two diluents.

8. The oral liquid pharmaceutical composition according to claim 7, wherein the diluent is the combination of propylene glycol and glycerin.

9. The oral liquid pharmaceutical composition according to claim 8, wherein the ratio of propylene glycol to glycerin is about 0.5:1 to 1:2.

10. The oral liquid pharmaceutical composition according to any preceding claims, further comprises sorbitol.

11. The oral liquid pharmaceutical composition according to claim 10, wherein sorbitol is in a concentration of 50 mg/mL to 250 mg/mL.

12. The oral liquid pharmaceutical composition according to any preceding claims, further comprises a buffer system selected from the group comprising sodium citrate, potassium citrate, sodium citrate dihydrate, citric acid, citric acid monohydrate, sodium bicarbonate, potassium bicarbonate, sodium dihydrogen phosphate and potassium dihydrogen phosphate and combination thereof.

13. The oral liquid pharmaceutical composition according to claim 12, wherein the buffer system is the combination of citric acid monohydrate and sodium citrate dihydrate.

14. The oral liquid pharmaceutical composition according to any preceding claims, further comprises at least one more herbal extract which is selected from the group comprising ginger, ginseng, licorice, echinacea, hedera helix and combination thereof.

## Patentansprüche

1. Orale flüssige pharmazeutische Zusammensetzung, umfassend Berberin oder eine pharmazeutisch akzeptable Salzform, Pelargonium sidoides, Propolis und mindestens einen pharmazeutisch akzeptablen Hilfsstoff.

2. Orale flüssige pharmazeutische Zusammensetzung gemäß Anspruch 1, wobei der pharmazeutisch akzeptable Hilfsstoff ein Verdünnungsmittel ist.

3. Orale flüssige pharmazeutische Zusammensetzung gemäß Anspruch 2, wobei die Gesamtkonzentration der Verdünnungsmittel etwa 50 mg/mL bis 900 mg/mL beträgt.

4. Orale flüssige pharmazeutische Zusammensetzung gemäß Anspruch 3, wobei die Gesamtkonzentration der Verdünnungsmittel etwa 100 mg/mL bis 800 mg/mL beträgt.

5. Orale flüssige pharmazeutische Zusammensetzung gemäß einem der vorhergehenden Ansprüche, wobei das Verdünnungsmittel aus der Gruppe ausgewählt ist, die Wasser, Dimethylisosorbid, Glykole, Propylenglykol, Ethylalkohol, Cetylalkohol, Glycerylstearat, Isopropylalkohol, Diethylamin, Glyceryloleat, Myristylalkohol, Gelatine, Cyclodextrin, Polyvinylpyrrolidon (Povidon), Benzylalkohol, Glycerin, Propylenglycol, Ethanol, Maltit, Xylit, Inosit, Mannit, Invertzucker, Sorbit und Kombinationen hiervon umfasst.

6. Orale flüssige pharmazeutische Zusammensetzung gemäß Anspruch 5, wobei das Verdünnungsmittel aus der Gruppe ausgewählt ist, die Glycerin, Propylenglycol, Sorbit, Wasser, Ethanol und Kombinationen hiervon umfasst.

7. Orale flüssige pharmazeutische Zusammensetzung gemäß einem der vorhergehenden Ansprüche, die mindestens zwei Verdünnungsmittel umfasst.

8. Orale flüssige pharmazeutische Zusammensetzung gemäß Anspruch 7, wobei das Verdünnungsmittel die Kombination von Propylenglycol und Glycerin ist.

9. Orale flüssige pharmazeutische Zusammensetzung gemäß Anspruch 8, wobei das Verhältnis von Propylenglycol zu Glycerin etwa 0,5:1 bis 1:2 beträgt.

10. Orale flüssige pharmazeutische Zusammensetzung gemäß einem der vorhergehenden Ansprüche, die des Weiteren Sorbit umfasst.

11. Orale flüssige pharmazeutische Zusammensetzung gemäß Anspruch 10, wobei Sorbit in einer Konzentration von 50 mg/mL bis 250 mg/mL vorliegt.

12. Orale flüssige pharmazeutische Zusammensetzung gemäß einem der vorhergehenden Ansprüche, die des Weiteren ein Puffersystem umfasst, das aus der Gruppe ausgewählt ist, die Natriumcitrat, Kaliumcitrat, Natriumcitrat-Dihydrat, Citronensäure, Citronensäure-Monohydrat, Natriumbicarbonat, Kaliumbicarbonat, Natriumdihydrogenphosphat und Kaliumdihydrogenphosphat und Kombination hiervon umfasst.

13. Orale flüssige pharmazeutische Zusammensetzung gemäß Anspruch 12, wobei das Puffersystem die Kombination aus Citronensäure-Monohydrat und Natriumcitrat-Dihydrat ist.

14. Orale flüssige pharmazeutische Zusammensetzung gemäß einem der vorhergehenden Ansprüche, die ferner mindestens ein weiteres Kräuterextrakt umfasst, das aus der Gruppe ausgewählt ist, die Ingwer, Ginseng, Süßholz, Echinacea, Hedera-Helix und Kombinationen hiervon umfasst.

## Revendications

1. Composition pharmaceutique liquide pour voie orale, comprenant de la berbérine ou une forme sel pharmacologiquement admissible, du pelargonium sidoides, de la propolis et au moins un excipient pharmacologiquement admissible.

2. Composition pharmaceutique liquide pour voie orale, conforme à la revendication 1, dans laquelle l'excipient pharmacologiquement admissible est un diluant.

3. Composition pharmaceutique liquide pour voie orale, conforme à la revendication 2, dans laquelle la concentration totale de diluants vaut à peu près de 50 mg/mL à 900 mg/mL.

4. Composition pharmaceutique liquide pour voie orale, conforme à la revendication 3, dans laquelle la concentration totale de diluants vaut à peu près de 100 mg/mL à 800 mg/mL.

5. Composition pharmaceutique liquide pour voie orale, conforme à l'une des revendications précédentes, dans laquelle le diluant est choisi dans l'ensemble comprenant eau, diméthyl-iso-sorbide, glycols, propylèneglycol, alcool éthylique, alcool cétylique, stéarate de glycéryle, alcool isopropylique, diéthylamine, oléate de glycéryle, alcool myristylique, gélatine, cyclodextrine, poly(vinylpyrrolidone) (Povidone), alcool benzylique, glycérine, propylèneglycol, éthanol, maltitol, xylitol, inositol, mannitol, sucres inversés, sorbitol, et leurs combinaisons.

6. Composition pharmaceutique liquide pour voie orale, conforme à la revendication 5, dans laquelle le diluant est choisi dans l'ensemble comprenant la glycérine, le propylèneglycol, le sorbitol, l'eau, l'éthanol et leurs combinaisons.

7. Composition pharmaceutique liquide pour voie orale, conforme à l'une des revendications précédentes, comprenant au moins deux diluants.

8. Composition pharmaceutique liquide pour voie orale, conforme à la revendication 7, dans laquelle le diluant est une combinaison de propylèneglycol et de glycérine.

9. Composition pharmaceutique liquide pour voie orale, conforme à la revendication 8, dans laquelle le rapport du propylèneglycol à la glycérine vaut à peu près de 0,5/1 à 1/2.

10. Composition pharmaceutique liquide pour voie orale, conforme à l'une des revendications précédentes, qui comprend en outre du sorbitol.

11. Composition pharmaceutique liquide pour voie orale, conforme à la revendication 10, dans laquelle le sorbitol se trouve en une concentration de 50 mg/mL à 250 mg/mL.

12. Composition pharmaceutique liquide pour voie orale, conforme à l'une des revendications précédentes, qui comprend en outre un système tampon choisi dans l'ensemble comprenant citrate de sodium, citrate de potassium, citrate de sodium dihydraté, acide citrique, acide citrique monohydraté, bicarbonate de sodium, bicarbonate de potassium, dihydrogénophosphate de sodium et dihydrogénophosphate de potassium, ainsi que leurs combinaisons.

13. Composition pharmaceutique liquide pour voie orale, conforme à la revendication 12, dans laquelle le système tampon est une combinaison d'acide citrique monohydraté et de citrate de sodium dihydraté.

14. Composition pharmaceutique liquide pour voie orale, conforme à l'une des revendications précédentes, qui comprend au moins un extrait végétal de plus, lequel est choisi dans l'ensemble comprenant les gingembre, ginseng, réglisse, echinacea, hedera hélix et leurs combinaisons.
